# EUROPEAN PATENT SPECIFICATION

(11) **EP 2 904 976 B1**
(45) Date of publication and mention of the grant of the patent: **24.04.2019**
(21) Application number: 13843120.0
(22) Date of filing: 27.09.2013
(51) Int. Cl.: A61B 10/02, A61B 10/04

(54) **BIOPSY INSTRUMENT**
BIOPSIEINSTRUMENT
INSTRUMENT POUR BIOPSIE

(30) Priority: 05.10.2012 US 201261710210 P
(43) Date of publication of application: 12.08.2015
(73) Proprietor: Olympus Corporation, Tokyo 192-8507 (JP)
(72) Inventor: SUZUKI, Keita, Tokyo 151-0072 (JP)
(74) Representative: Schicker, Silvia
(86) International application number: PCT/JP2013/076243
(87) International publication number: WO 2014/054525

(56) References cited:
- WO-A2-2010/138944
- JP-A- H11 262 490
- JP-A- S61 279 236
- JP-A- 2001 170 059
- JP-U- S4 874 687
- JP-U- S4 888 691
- US-A- 1 867 624
- US-A1- 2012 004 573

## Description

### Technical Field

The present disclosure relates generally to a biopsy instrument configured to collect body tissue, and more particularly, to a biopsy instrument inserted into a treatment tool channel of an endoscope and used therein. Priority is claimed on US Provisional Patent Application No. 61/710,210, filed on October 5, 2012.

### Background Art

In the related art, an inspection method known as a biopsy, in which a small amount of body tissue is collected and the body tissue is observed using a microscope, is known. As a biopsy instrument configured to endoscopically collect the body tissue used for a biopsy, for example, Patent Document 1 discloses a biopsy instrument. The biopsy instrument includes a distal end tip, a manipulation wire connected to the distal end tip, and a flexible tube having a distal end member formed at a distal end thereof and through which the manipulation wire is inserted. A hole having an open surface is formed at a proximal end side of the distal end tip, and a sharp cutting blade is formed throughout the entire circumferential edge of the hole. When the distal end tip is inserted into the tissue and then is used to pull the manipulation wire to retract the distal end tip, a portion of the tissue is sandwiched between the cutting blade and the distal end member to be cut and accommodated in a tissue piece collecting hole. Accordingly, the biopsy instrument is capable of collecting the body tissue.

Document US 2012/0004573 A1 describes a biopsy collection apparatus comprising a cannula which surrounds an inner needle, wherein the inner needle and the cannula are configured to move relative to one another for the collection of a tissue sample. The cannula is hollow and substantially cylindrical in shape, and comprises a proximal end with a cutting edge that is configured to cut through the tissue of a target organ. Once the apparatus is properly positioned, a releasable lock is triggered, and a biased spring of the cannula propels the cutting edge through a stomach wall, toward a tip of the needle, wherein as the cannula passes through the stomach wall and advances past a notch portion, a full-thickness biopsy is removed from stomach wall.

In document WO 2010/138944 A2, a biopsy device needle set is described that comprises an inner cannula having two longitudinal linkages linking an inner cannula body to an inner cannula proximal end, and an outer cannula including two linkages linking an outer cannula body to its proximal end. When the needle set is loaded into a biopsy device and deployed to a tissue site, a cutting edge of outer cannula extends into and cuts through the tissue, capturing a volume of tissue circumscribed by the cutting edge into the lumen of inner cannula, and as the needle set reaches its full extension into the tissue site, the outer cannula is rotated about its axis, causing proximal end of inner cannula to rotate relative to inner cannula body, which in turn twists inner cannula linkages until they meet, pinching tissue through which linkages pass along the way.

Document US 1 867 624 A discloses a device for obtaining specimens of subcutaneous tissues comprising a non-slotted tube having a cutting edge and a cutting rod capable of longitudinal movement within and removable from the tube. The cutting rod has an extended conical head capable of extension beyond the extremity of the tube and of longitudinal movement within the tube, the base of said head being recessed to form a peripheral cutting edge co-operating with the cutting edge of said tube during the longitudinal movement of the cutting rod to produce a shearing effect.

Document JP S61 279236 describes a specimen extraction device for body tissue, which comprises an annular pointed head having a recess with a sharp edge.

### Citation List

### Patent Literature

Patent Document 1: Japanese Unexamined Patent Application, First Publication No. S61-279236

### Summary of invention

### Problem to be Solved by Invention

Actually, it is not easy to collect body tissue by using the biopsy instrument disclosed in the Patent Document 1. In the biopsy instrument disclosed in the Patent Document 1, since cutting blades are provided over a peripheral edge of an opening surface of a hole, a contact area between the cutting blades and the body tissue is large. For this reason, for example, a situation that a large force is required to be applied to cut the tissue, or a situation that the tissue interposed between the cutting blades and the distal-end members slips such that the tissue cannot be favorably cut off is considered. Therefore, a biopsy instrument more easily to use is desired.

The present invention has been made in consideration of such circumstances and an object of the present invention is to provide a biopsy instrument to cut and collect body tissue certainly through an easy manipulation.

### Means for Solving Problem

The present invention concerns a biopsy instrument having the features of claim 1.

According to a first aspect of the present invention, the biopsy instrument includes a needle pipe formed in a tubular shape having a convex section, the convex section having a sharp end section, the convex section being provided at a distal end section of the needle pipe, a distal end tip provided so as to be capable of advancing and retracting with respect to the needle pipe, a manipulation member connected to the distal end tip and inserted into the needle pipe, and a cutting blade section provided at a proximal end side of the distal end tip and having a sharp end section.

According to a second aspect of the present invention, in the biopsy instrument according to the first aspect, the needle pipe may have at least two convex sections, and a number of the cutting blade sections may be the same as a number of the convex sections.

According to a third aspect of the present invention, in the biopsy instrument according to the first aspect, the end section of the cutting blade section may be disposed between the plurality of the convex sections in a circumferential direction of the needle pipe. The plurality of the convex sections and the plurality of the cutting blade sections may be alternately disposed in the circumferential direction of the needle pipe.

According to a fourth aspect of the present invention, in the biopsy instrument according to the first aspect, the distal end tip may have a sharp puncturing section formed at a distal end side of the distal end tip.

According to a fifth aspect of the present invention, in the biopsy instrument according to the first aspect, the width of a distal end side of the needle pipe may be set such that the distal end side of the needle pipe is capable of entering the distal end tip.

### Advantageous Effects of Invention

According to the above-mentioned biopsy instrument, body tissue can be certainly cut and collected through an easy manipulation.

### Brief Description of Drawings

FIG. 1 is a view showing the overall configuration of a biopsy instrument according to a first embodiment of the present invention;
FIG. 2A is an enlarged view showing a periphery of a distal end section of a needle pipe of the biopsy instrument according to the first embodiment of the present invention;
FIG. 2B is a view when seen in a direction of an arrow A of FIG. 2A;
FIG. 3 is a cross-sectional view of the distal end section of the biopsy instrument according to the first embodiment of the present invention;
FIG. 4 is a view showing a process upon use of the biopsy instrument according to the first embodiment of the present invention;
FIG. 5 is a view showing a process upon use of the biopsy instrument according to the first embodiment of the present invention;
FIG. 6 is a view showing a process upon use of the biopsy instrument according to the first embodiment of the present invention; and
FIG. 7 is an enlarged view showing a periphery of a distal end section of a needle pipe of a biopsy instrument according to a second embodiment of the present invention.

### Description of Embodiments

### (First Embodiment)

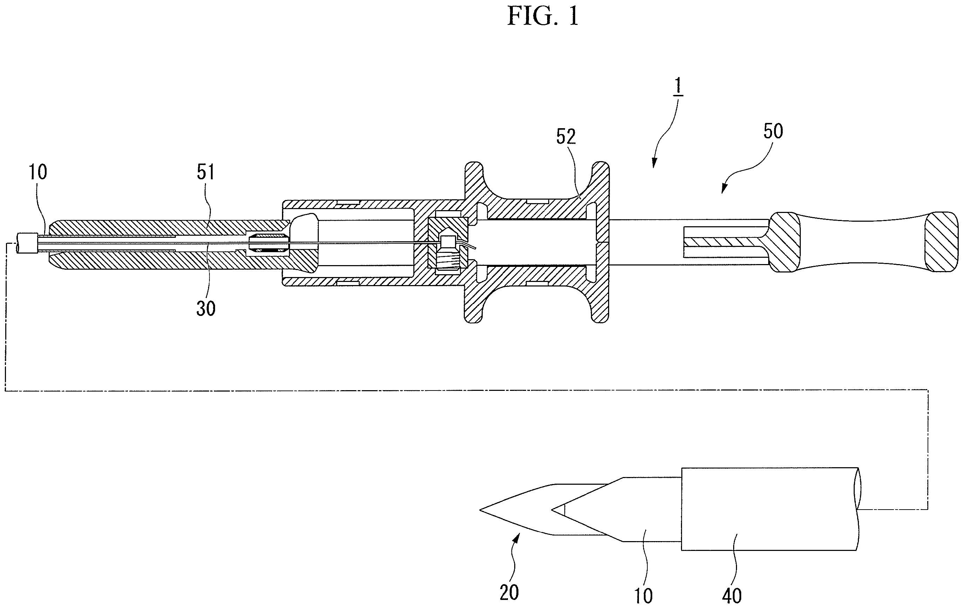

Hereinafter, a first embodiment of a biopsy instrument will be described with reference to FIGS. 1 to 6.

FIG. 1 is a view showing an overall configuration of a biopsy instrument 1 of the first embodiment. The biopsy instrument 1 includes a long needle pipe 10 having flexibility, a distal end tip 20, a sheath 40, and a manipulation section (a manipulation member) 50. The distal end tip 20 is inserted so as to be capable of advancing and retracting with respect to the needle pipe 10. The needle pipe 10 is inserted into the sheath 40 so as to capable of advancing and retracting. The manipulation section 50 is attached to a proximal end section of the needle pipe 10.

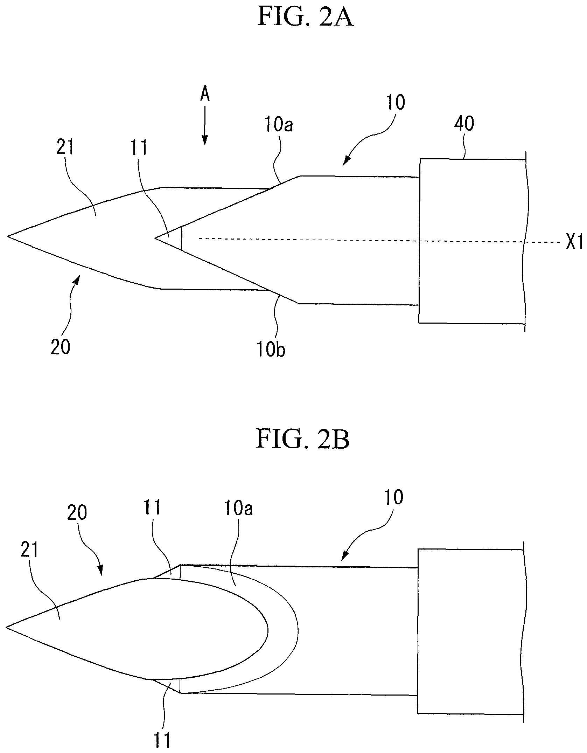

FIG. 2A is an enlarged view showing a periphery of the distal end section of the needle pipe 10. FIG. 2B is a view when seen in a direction of an arrow A shown in FIG. 2A. The needle pipe 10 is formed of a metal such as stainless steel or the like in a tubular shape having a lumen. The needle pipe 10 has a small outer diameter of, for example, about 1 to 2 millimeters (mm) and also has flexibility. As shown in FIGS. 2A and 2B, the needle pipe 10 has two convex sections 11 having sharp end sections 11a disposed at the distal end of the needle pipe 10. The two convex sections 11 are formed by cutting a portion of the member that forms the needle pipe 10 such that distal end surfaces of the needle pipe 10 are inclined with respect to an axis XI of the needle pipe 10, and have inclined surfaces 10a and 10b which approach an outer circumferential surface of the needle pipe 10 as it goes toward the proximal end of the needle pipe 10.

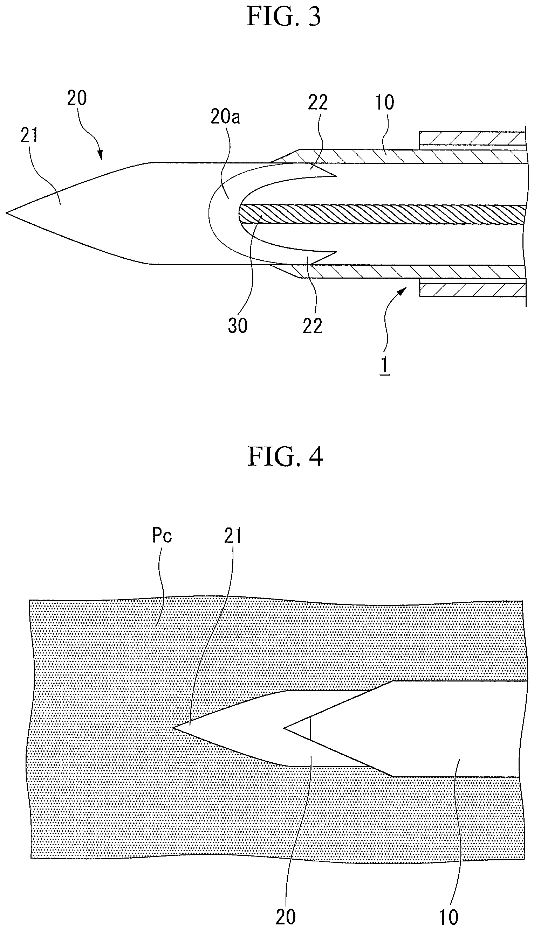

FIG. 3 is a cross-sectional view of the distal end section of the biopsy instrument 1. The distal end tip 20 has a sharp puncturing section 21 with a substantially conical shape and being disposed at the distal end side of the distal end tip 20. The proximal end side of the distal end tip 20 is formed in a substantially cylindrical shape having a lumen. Since an outer diameter of the distal end tip 20 is smaller than the inner diameter of the needle pipe 10, as shown in FIG. 3, the distal end tip 20 is capable of entering the needle pipe 10.

Inclined surfaces 20a and ((not shown but substantially similar to inclined surface 20a and offset by 180 degrees from inclined surface 20a) are formed at the proximal end side of the distal end tip 20 through the same processing as the distal end section of the needle pipe 10. Two cutting blade sections 22 having sharp end sections are formed at end sections of the inclined surfaces 20a and 20b. A manipulation wire (a manipulation member) 30 is connected to the distal end tip 20 in the lumen by welding or the like. The manipulation wire 30 extends to the manipulation section 50 through the inside of the needle pipe 10 and is configured to be capable of advancing and retracting with respect to the needle pipe 10.

Positions of the distal end tip 20 and the needle pipe 10 are determined in a way that the convex section 11 and the cutting blade section 22 are disposed to be alternately positioned in a circumferential direction of the needle pipe 10. In the first embodiment, the two convex sections 11 are disposed at positions separated from each other by 180 degrees in the circumferential direction of the needle pipe 10. The two cutting blade sections 22 are disposed at positions separated from each other by 180 degrees in the circumferential direction of the distal end tip 20. A position relationship between the distal end tip 20 and the needle pipe 10 is defined such that each of the cutting blade sections 22 is disposed at a position separated from each of the convex sections in the circumferential direction of the needle pipe 10 by 90 degrees.

The sheath 40 may be appropriately selected from known sheaths having flexibility and through which the needle pipe 10 is capable of being inserted to advance and retract. The material of the sheath 40 is not particularly limited either and various known materials such as a resin, a coil, or the like, may be used.

As shown in FIG. 1, the manipulation section 50 includes a manipulation section main body 51 which is fixed to the proximal end section of the needle pipe 10 and a slider 52 which is attached to be slidable in a longitudinal direction of the manipulation section main body 51. A basic configuration of the manipulation section 50 is known. The proximal end section of the manipulation wire 30 extending through the inside of the needle pipe 10 protrudes into the internal space of the manipulation section main body 51 to be fixed to the slider 52. Accordingly, as the slider 52 is slid with respect to the manipulation section main body 51, the manipulation wire 30 is capable of advancing and retracting with respect to the needle pipe 10. When the manipulation section main body 51 is relatively moved with respect to the sheath 40, the needle pipe 10 and the distal end tip 20 is capable of advancing and retracting with respect to the sheath 40. Accordingly, the manipulation section 50 is capable of adjusting the protrusion amount of the needle pipe 10 and the distal end tip 20 protruding from the distal end of the sheath 40.

In an operation using the biopsy instrument 1 of the first embodiment having the above-mentioned configuration, the example in which the pancreas is a tissue of a biopsy target (hereinafter, simply referred to as a "target tissue") will be described.

An operator first introduces an endoscope (not shown) into a patient's body and moves a distal end section of the endoscope to the vicinity of the pancreas. Next, in a state in which the needle pipe 10 and the distal end tip 20 are accommodated in the sheath 40, the operator inserts the biopsy instrument 1 into a forceps channel of the endoscope from the puncturing section 21 side, and causes the distal end section of the sheath 40 to protrude from the distal end of the forceps channel. The endoscope may be appropriately selected from various known endoscopes such as an optical endoscope, an ultrasonic endoscope, and so on, according to the kind or the position of the target tissue.

The operator manipulates the manipulation section main body 51 while checking the pancreas and a portion of the pancreas from which the tissue piece is collected (a collecting area) within a field of vision of the endoscope, and causes the needle pipe 10 and the distal end tip 20 to be protruded from the sheath 40. Further, as shown in FIG. 4, the operator performs manipulation of inserting the puncturing section 21 into the pancreas Pc and advancing the puncturing section 21 to a position slightly in front of the collecting area by, for example, several millimeters.

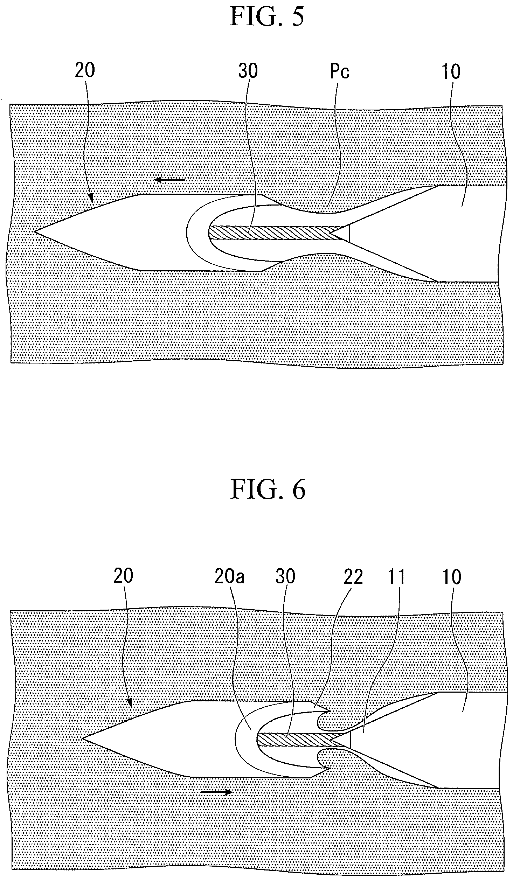

Next, when the operator moves the slider 52 forward with respect to the manipulation section main body 51, the manipulation wire 30 connected to the slider 52 moves forward with respect to the needle pipe 10, and as shown in FIG. 5, the distal end tip 20 protrudes in front of the needle pipe 10. Accordingly, the distal end tip 20 is separated from the needle pipe 10, and a portion of the tissue of the pancreas Pc enters a space generated between the distal end tip 20 and the needle pipe 10.

When the operator retracts the slider 52 with respect to the manipulation section main body 51 in this state, as shown in FIG. 6, the distal end tip 20 is retracted to approach the needle pipe 10. Here, the cutting blade section 22 of the retracted distal end tip 20 presses and gradually cuts the tissue entering between the distal end tip 20 and the needle pipe 10. An area of the entered tissue disposed between the two cutting blade sections 22 in the circumferential direction of the distal end tip 20 is hard for the distal end tip 20 to cut. However, when the distal end tip 20 approaches the needle pipe 10, the convex section 11 of the needle pipe 10 presses the area such that the area is sandwiched between the convex section 11 and the distal end tip 20. As a result, the area is gradually cut by the convex section 11 of the needle pipe 10 disposed at an opposite side of the distal end tip 20. When the distal end tip 20 is retracted until the inclined surface 20a formed at the proximal end side of the distal end tip 20 and the other inclined surface 20b (not shown) are disposed in the lumen of the needle pipe 10, and the portion of the tissue is cut, separated and accommodated in the needle pipe 10 as a tissue piece.

According to the biopsy instrument 1 of the first embodiment, the two convex sections 11 having the sharp distal ends are disposed at the needle pipe 10, and the two the cutting blade sections 22 having the sharp distal ends are also disposed at the distal end tip 20. For this reason, when the distal end tip 20 approaches the needle pipe 10 to gradually cut the tissue, a force applied to the tissue is capable of being concentrated on the convex section 11 and the cutting blade section 22 to appropriately cut the tissue. As a result, the body tissue is capable of being securely cut and collected with easy manipulation through retraction of the slider 52.

In addition, according to the biopsy instrument 1 of the first embodiment, the convex sections 11 and the cutting blade sections 22 are alternately disposed in the circumferential direction of the needle pipe 10. For this reason, an area of the tissue that is hard for the convex section 11 to cut is cut by the cutting blade section 22, and an area of the tissue that is hard for the cutting blade section 22 to cut is cut by the convex section 11. As a result, separation and collection of the body tissue is capable of being more appropriately performed.

In the flow of the above-mentioned procedure, an example in which the distal end tip is moved forward with respect to the needle pipe by advancing the puncturing section to a position slightly in front of the collecting area has been described. However, instead of this, a procedure of advancing the puncturing section to the collecting area or a position slightly behind the collecting area and then retracting the needle pipe with respect to the distal end tip may be used. The distal end tip is capable of being separated from the needle pipe in this manipulation as well. As a result, as the distal end tip approaches the needle pipe, cutting and collecting of the tissue is capable of being performed in the same manner.

### (Second Embodiment)

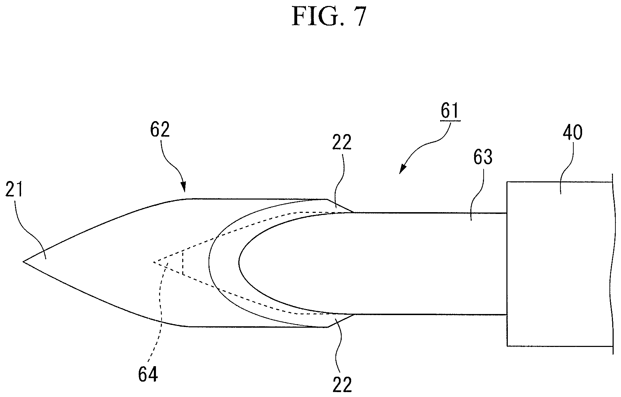

Next, a second embodiment will be described with reference to FIG. 7. A biopsy instrument 61 according to the second embodiment is distinguished from the above-mentioned biopsy instrument 1 in that a size relation between the distal end tip and the needle pipe is varied. Further, in the following description, the same elements as the above-mentioned second embodiment are designated by the same reference numerals and an overlapping description thereof will be omitted here.

FIG. 7 is an enlarged view of a periphery of a distal end section of a needle pipe 63 in the biopsy instrument 61. In the biopsy instrument 61, as shown in FIG. 7, the inner diameter of a distal end tip 62 near a proximal end is set to be larger than the outer diameter of the needle pipe 63, and a distal end side of the needle pipe 63 including a convex section 64 is configured such that the distal end side of the needle pipe 63 is capable of entering the proximal end side of the distal end tip 62. The other aspects are substantially the same as those of the biopsy instrument 1 according to the first embodiment.

In the biopsy instrument 61 according to the present second embodiment, similar to that of the first embodiment, the distal end tip 62 is capable of approaching the needle pipe 63 through easy manipulation by simply extracting the slider 52 to securely cut and collect the body tissue.

In addition, when the needle pipe 63 collides with the distal end tip 62, the distal end tip 62 is disposed not to retract further with respect to the needle pipe 63. For this reason, even if a reaction force is applied to the distal end tip 62 from the tissue when the puncturing section 21 is inserted into the target tissue, the puncturing section 21 does not enter the needle pipe 63. Accordingly, there is no need to hold the slider 52 such that the puncturing section 21 does not enter the needle pipe 63 upon insertion of the target tissue, and the manipulation is capable of being further facilitated.

Hereinabove, while the above embodiments have been described, the technical spirit of the present invention is not limited to the embodiments, and combinations of the components may be varied or various modifications may be added to or deleted from the components in the embodiments without departing from the spirit of the present invention.

For example, the number of convex sections may be equal to the number of cutting blade sections. For example, neither of the number of convex sections or the number of cutting blade sections is limited to 2 as described in the above-mentioned embodiment, and each of the number of convex sections and the number of cutting blade sections may be 3 or more. However, as the number of convex sections and the number of cutting blade sections are increased, a force applied to the tissue is distributed. For this reason, the skill required to manipulate the distal end tip to approach the needle pipe is increased. Accordingly, both the number of convex sections and the number of cutting blade sections is most preferably 2.

Further, a certain effect is capable of being obtained even when only one of each of the convex section and the cutting blade section is provided or the number of convex sections is different from the number of cutting blade sections.

### Industrial Applicability

According to the biopsy instrument, it is possible to provide a biopsy instrument to certainly cut and collect body tissue through an easy manipulation.

### Reference Signs List

- 1, 61: biopsy instrument
- 10, 63: needle pipe
- 11, 64: convex section
- 20, 62: end tip
- 22: cutting blade section
- 30: manipulation wire (a manipulation member)

## Claims

1. A biopsy instrument (1, 61) comprising:
a needle pipe (10, 63) formed in a tubular shape, wherein the needle pipe (10, 63) has a plurality of convex sections (11, 64), each convex section having a sharp end section and being provided at a distal end section of the needle pipe (10, 63);
a distal end tip (20, 62) provided so as to be capable of advancing and retracting with respect to the needle pipe (10, 63);
a manipulation member (30) connected to the distal end tip (20, 62) and inserted into the needle pipe (10, 63); and
cutting blade sections (22), each being provided at a proximal end side of the distal end tip (20, 62) and having a sharp end section,
wherein a number of the cutting blade sections (22) is the same as a number of the convex sections (11, 64).

2. The biopsy instrument (1) according to claim 1,
wherein the end section of each cutting blade section (22) is disposed between the plurality of the convex sections (11, 64) in a circumferential direction of the needle pipe (10, 63), and
the plurality of the convex sections and the plurality of the cutting blade sections (22) are alternately disposed in the circumferential direction of the needle pipe (10).

3. The biopsy instrument according to claim 1, wherein the distal end tip (20, 62) comprises a sharp puncturing section (21) formed at a distal end side of the distal end tip (20, 62).

4. The biopsy instrument according to claim 1, wherein a width of a distal end side of the needle pipe (10, 63) is set such that the distal end side of the needle pipe (10, 63) is capable of entering the distal end tip (20, 62).

## Patentansprüche

1. Biopsieinstrument (1, 61), umfassend:
ein Nadelrohr (10, 63), das in einer rohrförmigen Form gebildet ist, wobei das Nadelrohr (10, 63) eine Mehrzahl konvexer Abschnitte (11, 64) aufweist, wobei jeder konvexe Abschnitt einen scharfen Endabschnitt aufweist und an einem Distalendabschnitt des Nadelrohrs (10, 63) vorgesehen ist;
eine Distalendspitze (20, 62), die derart vorgesehen ist, dass sie mit Bezug auf das Nadelrohr (10, 63) vorgeschoben und zurückgezogen werden kann;
ein Manipulationselement (30), das mit der Distalendspitze (20, 62) verbunden ist und in das Nadelrohr (10, 63) eingesetzt ist; und
Schneidmesserabschnitte (22), die jeweils an einer Proximalendseite der Distalendspitze (20, 62) vorgesehen sind und einen scharfen Endabschnitt aufweisen,
wobei eine Anzahl der Schneidmesserabschnitte (22) dieselbe ist wie eine Anzahl der konvexen Abschnitte (11, 64).

2. Biopsieinstrument (1) nach Anspruch 1,
wobei der Endabschnitt eines jeden Schneidmesserabschnitts (22) zwischen der Mehrzahl der konvexen Abschnitte (11, 64) in einer Umfangsrichtung des Nadelrohrs (10, 63) angeordnet ist, und
die Mehrzahl der konvexen Abschnitte und die Mehrzahl der Schneidmesserabschnitte (22) abwechselnd in der Umfangsrichtung des Nadelrohrs (10) angeordnet sind.

3. Biopsieinstrument nach Anspruch 1, wobei die Distalendspitze (20, 62) einen scharfen Punktionsabschnitt (21) umfasst, der auf einer Distalendseite der Distalendspitze (20, 62) gebildet ist.

4. Biopsieinstrument nach Anspruch 1, wobei eine Breite einer Distalendseite des Nadelrohrs (10, 63) derart eingestellt ist, dass die Distalendseite des Nadelrohrs (10, 63) die Distalendspitze (20, 62) aufnehmen kann.

## Revendications

1. Instrument pour biopsie (1, 61) comprenant :
un tube d'aiguille (10, 63) réalisé en une forme tubulaire, le tube d'aiguille (10, 63) ayant une pluralité de sections convexes (11, 64), chaque section convexe ayant une section d'extrémité coupante et étant prévue à une section d'extrémité distale du tube d'aiguille (10, 63) ;
une pointe d'extrémité distale (20, 62) prévue de façon à être capable d'avancer et de reculer par rapport au tube d'aiguille (10, 63) ;
un élément de manipulation (30) relié à la pointe d'extrémité distale (20, 62) et introduit dans le tube d'aiguille (10, 63) ; et
des sections de lame de coupe (22), chacune étant prévue à un côté extrémité proximale de la pointe d'extrémité distale (20, 62) et ayant une section d'extrémité coupante,
un nombre des sections de lame de coupe (22) étant le même qu'un nombre des sections convexes (11, 64).

2. Instrument pour biopsie (1) selon la revendication 1,
dans lequel la section d'extrémité de chaque section de lame de coupe (22) est disposée entre la pluralité des sections convexes (11, 64) dans une direction circonférentielle du tube d'aiguille (10, 63), et
la pluralité de sections convexes et la pluralité de sections de lame de coupe (22) sont disposées de manière alternée dans la direction circonférentielle du tube d'aiguille (10).

3. Instrument pour biopsie (1) selon la revendication 1, dans lequel la pointe d'extrémité distale (20, 62) comprend une section de perforation coupante (21) formée à un côté extrémité distale de la pointe d'extrémité distale (20, 62).

4. Instrument pour biopsie (1) selon la revendication 1, dans lequel une largeur d'un côté extrémité distale du tube d'aiguille (10, 63) est définie de telle sorte que le côté extrémité distale du tube d'aiguille (10, 63) est capable d'entrer dans la pointe d'extrémité distale (20, 62).
